# EUROPÄISCHE PATENTSCHRIFT

(11) **EP 1 841 461 B1**
(45) Veröffentlichungstag und Bekanntmachung des Hinweises auf die Patenterteilung: **14.04.2010**
(21) Anmeldenummer: 06706393.3
(22) Anmeldetag: 25.01.2006
(51) Int. Cl.: A61K 48/00

(54) **INIIZIERBARES MITTEL ZUR ZIELGERICHTETEN BEHANDLUNG VON RETINALEN GANGLIENZELLEN**
INJECTABLE AGENT FOR THE TARGETED TREATMENT OF RETINAL GANGLION CELLS
SUBSTANCE INJECTABLE POUR LE TRAITEMENT CIBLE DE CELLULES GANGLIONNAIRES DE LA RETINE

(30) Priorität: 30.01.2005 DE 102005005528
(43) Veröffentlichungstag der Anmeldung: 10.10.2007
(73) Patentinhaber: Eberhard-Karls-Universität Tübingen, 72076 Tübingen (DE)
(72) Erfinder: THALER, Sebastian, 72076 Tübingen (DE); SCHÜTTAUF, Frank, 72076 Tübingen (DE)
(74) Vertreter: Findeisen, Marco
(86) Internationale Anmeldenummer: PCT/EP2006/000622
(87) Internationale Veröffentlichungsnummer: WO 2006/079508

(56) Entgegenhaltungen:
- US-B1- 6 200 801
- GARCIA VALENZUELA E ET AL: "Rescue of retinal ganglion cells from axotomy-induced apoptosis through TRK oncogene transfer" NEUROREPORT, Bd. 9, Nr. 14, 5. Oktober 1998 (1998-10-05), Seiten 3165-3170, XP008064060 ISSN: 0959-4965
- CAYOUETTE MICHEL ET AL: "Adenovirus-mediated gene transfer to retinal ganglion cells" INVESTIGATIVE OPHTHALMOLOGY AND VISUAL SCIENCE, Bd. 37, Nr. 10, 1996, Seiten 2022-2028, XP002380396 ISSN: 0146-0404
- ISENMANN S ET AL: "BAX ANTISENSE OLIGONUCLEOTIDES REDUCE AXOTOMY-INDUCED RETINAL GANGLION CELL DEATH IN VIVO BY REDUCTION OF BAX PROTEIN EXPRESSION" CELL DEATH AND DIFFERENTIATION, EDWARD ARNOLD, OXFORD, GB, Bd. 6, Nr. 7, Juli 1999 (1999-07), Seiten 673-682, XP008013601 ISSN: 1350-9047
- GARCIA-VALENZUELA E ET AL: "Axon-mediated gene transfer of retinal ganglion cells in vivo" JOURNAL OF NEUROBIOLOGY, Bd. 32, Nr. 1, 1997, Seiten 111-122, XP002380395 ISSN: 0022-3034
- REJDAK ROBERT ET AL: "Age-dependent decrease of retinal kynurenate and kynurenine aminotransferases in DBA/2J mice, a model of ocular hypertension." VISION RESEARCH, Bd. 44, Nr. 7, März 2004 (2004-03), Seiten 655-660, XP002380469 ISSN: 0042-6989
- THALER S ET AL: "Downregulation of KAT II in retinal ganglion cells via intravitreal or retrograde delivery of antisense oligonucleotides" IOVS, Bd. 46, Nr. Suppl. S, 2005, Seite 1317, XP002380491 & ANNUAL MEETING OF THE ASSOCIATION-FOR-RESEARCH-IN-VISION-AND-OPH THALM OLOGY; FT LAUDERDALE, FL, USA; MAY 01 -05, 2005 ISSN: 0146-0404
- THALER SEBASTIAN ET AL: "A selective method for transfection of retinal ganglion cells by retrograde transfer of antisense oligonucleotides against kynurenine aminotransferase II." MOLECULAR VISION [ELECTRONIC RESOURCE]. 2006, Bd. 12, 2006, Seiten 100-107, XP002380492 ISSN: 1090-0535

## Beschreibung

Die vorliegende Erfindung betrifft die zielgerichtete Behandlung von retinalen Ganglienzellen (engl.: retinal ganglion cells, RGC).

Die zielgerichtete Behandlung von Erkrankungen der Retina, wie bspw. Glaukom, Retinopathia Diabetica, stellt eine der großen Herausforderungen an die Medizin und Arzneimittelforschung im Bereich der Ophthalmologie, d.h, der modernen Augenheilkunde, dar. Dabei geht es insbesondere darum, eine Substanz oder ein Mittel bereitzustellen, das in die retinalen Ganglienzellen, d.h. in die Nervenzellen des Wirbeltierauges, deren Axone das Auge über den Sehnerv verlassen, gelangt. Wichtig ist in diesem Zusammenhang, dass eine solche Substanz oder ein solches Mittel ausschließlich und zielgerichtet in die retinalen Ganglienzellen gelangt und dort ggf. seine Aktivität entfalten kann, nicht aber in anderen Bereichen des Auges bzw. der Retina. Nur so ist eine selektive Behandlung der Erkrankung von retinalen Ganglienzellen möglich, ohne dass andere Gewebe bzw. Zellen des Organismus oder Auges in ihrer physiologischen Funktion beeinträchtigt oder beschädigt werden. Nebenwirkungen einer entsprechenden Behandlung eines Patienten werden dadurch reduziert.

Shen et al. (2002), "Preclinical evaluation of a phosphorothioate oligonucleotide in the retina of rhesus monkey", Lab. Invest. 82, Seiten 167 bis 182, beschreiben ein Oligonucleotid, das in den *Corpus vitreum,* d.h. den Glaskörper des Auges, oder in subretinale Bereiche injizierbar ist und dadurch in die Retina eingebracht werden kann.

Ähnliche Daten über Experimente, in denen direkt in die Augen injizierbare Substanzen beschrieben werden, wurden bereits zuvor von Shen, W. Y. und Rakoczy, W. E. (2001), "Uptake dynamics and retinal tolerance of phosphorothioate oligonucleotide and its direct delivery into the site of choroidal neovascularization through subretinal administration in the rat", Antisense Nucleic Acid Drug Dev. 11, Seiten 257 bis 264, und Vorwerk et al. (2000), "Depression of retinal glutamate transporter function leads to elevated intravitreal glutamate levels and ganglion cell death", Invest Ophthalmol. Vis. Sei. 41, Seiten 3615 bis 3621, präsentiert.

Isenmann et al. (1999), "Bax antisense oligonucleotides reduce axotomy-induced retinal ganglion cell death in vivo by reduction of Bax protein expression", Cell Dead and Differentiation 6, Seiten 673-682, beschreiben die intravitreale Injektion eines Antisense-Oligonucleotides, das in den retinalen Ganglien-Zellen (RGC) die Expression des Bax-Proteins inhibiert.

Injektionen in das Auge, speziell in den *Corpus vitreum,* die auch als intravitreale Injektionen bezeichnet werden, sind jedoch nachteilig. So besteht bei einer solchen Injektion die Gefahr, dass die Linse des Auges massiv beschädigt wird. Bei dieser Applikation kann es zu Blutungen an der Einstichstelle oder zu Infektionen kommen, die das gesamte Auge betreffen. Ferner besteht die Gefahr, dass die Linse des Auges beschädigt wird, was in der Folge zu einer Modulation neuroprotektiver Stoffwechselwege führen kann, was Untersuchungen und Therapien in diesem Bereich massiv beeinträchtigt oder gänzlich unmöglich macht.

Ein weiterer ganz entscheidender Nachteil der intravitreal injizierbaren Substanzen, wie Oligonucleotiden, besteht darin, dass diese nach ihrer Injektion unspezifisch in mehr oder weniger sämtliche retinalen Zellschichten und andere Regionen des Organismus transportiert werden und deshalb ein selektiver Transport der Substanzen zu den retinalen Ganglienzellen nicht möglich ist. Intravitreal injizierbare Substanzen sind deshalb für eine zielgerichtete Behandlung von Erkrankungen der retinalen Ganglienzellen ungeeignet.

Husak et al. (2000), "Pseudorabies virus membrane proteins gI and gE facilitate anterograde spread of infection in projection-specific neurons in the rat", J. Virol. 74, Seiten 10975 bis 10983, beschreiben Viruspartikel bzw. virale Vektoren, die in Bereiche des Mittelhirns, d.h. in den *Colliculus superior,* injizierbar sind und über einen sich daran anschließenden retrograden Transport im Gewebe des Gehirns bis hin zur Retina bzw. zu den retinalen Ganglienzellen gelangen.

Vergleichbare Viruspartikel oder virale Konstrukte werden von Kaspar et al. (2002), "Targeted retrograde gene delivery for neuronal protection", Mol. Ther. 5, Seiten 50 bis 56, und von Peltekian et al. (2002), "Neurotropism and retrograde axonal transport of a canine adenoviral vector: a tool for targeting key structures undergoing neurodegenerative processes", Mol. Ther. 5, Seiten 25 bis 32, beschrieben.

Virale Vektoren und sich davon ableitende Plasmide, die in Bereiche des Mittelhirns appliziert werden, sind beispielsweise beschrieben in Valenzuela und Sharma (1998), "Rescue of retinal ganglion cells from axatomy-induced apoptosis through TRK oncogene transfer", NeuroReport 9, Seiten 3165-3170; Cayouette und Gravel (1996), "Adenovirus-mediated gene transfer to retinal ganglion cells", Investigative Ophthalmology & Visual Science, 37, Seiten 2022-2028; Garcia-Valenzuela et al. (1997), "Axonmediated gene transfer of retinal ganglion cells in vivo", J. Neurobiol 32, Seiten 111-122; und in der US 6,200,801.

Virale Vektoren sind jedoch für eine therapeutische oder diagnostische Anwendung äußerst problematisch, da diese nach Einbringung in den Organismus eine Vielzahl von virusspezifischen Nebenwirkungen hervorrufen, die bis zur Induktion der Apoptose reichen können. Ferner ist die Herstellung derartiger viraler Konstrukte mit hohen Kosten und größerem Aufwand verbunden, so dass eine großmaßstabliche Anwendung nicht in Frage kommt. Virale Vektoren und Viruspartikel sind deshalb für eine zielgerichtete Behandlung von Erkrankungen der Retina ebenfalls nicht geeignet.

Aufgabe der vorliegenden Erfindung ist es deshalb, ein in das Gehirn eines Lebewesens injizierbares RGC-spezifisches Mittel bereitzustellen, mit dem die Nachteile aus dem Stand der Technik vermieden werden. Insbesondere soll ein solches Mittel bereitgestellt werden, das therapeutisches bzw. diagnostisches Potenzial aufweist und zielgerichtet in retinale Ganglienzellen transportiert werden kann. Dieses Mittel soll einfach herstellbar sein und sich nach einer Injektion in das Gehirn eines Lebewesens hauptsächlich in den retinalen Ganglienzellen und nicht oder nur in geringem zu tolerierendem Maße in anderen retinalen Zellschichten oder anderen Zellen des Organismus akkumulieren.

Diese Aufgabe wird durch die Verwendung eines Antisense-Oligonucleotids zur Herstellung eines Mittels zur Behandlung einer Erkrankung des Auges, die mit einer Genüberexpression in den retinalen Ganglienzellen assoziiert ist, zur Injektion in den *Colliculus superior* gelöst, so dass das Nucleinsäuremolekül zielgerichtet in retinale Ganglienzellen transportiert wird und dort die Genexpression inhibiert, wobei das Antisense-Oligonucleotid eine Nucleotidsequenz aufweist, die komplementär zu der Sequenz einer mRNA aus retinalen Ganglienzellen ist.

Die Erfinder haben nämlich überraschenderweise unter Zuhilfenahme eines Rattenmodells herausgefunden, dass ein isoliertes, Nucleinsäuremolekül, bzw. ein Antisense-Oligonucleotid, nach der Injektion in das Gehirn eines Säuretieres zielgerichtet in retinale Ganglienzellen transportiert wird. Dabei war besonders überraschend, dass ein solches Nucleinsäuremolekül keinerlei virale Komponenten benötigt, um mittels retrogradem Transport zu den RGC zu gelangen. Erstaunlich war die Beobachtung, dass sich ein solches Nucleinsäuremolekül ausschließlich in den RGC wiederfindet, in anderen retinalen Zellschichten oder sonstigen Zellen des Organismus hingegen keinerlei Nucleinsäuremoleküle nach der Injektion des letzteren in das Gehirn nachweisbar waren.

Ein solches Ergebnis war nicht zu erwarten. Vielmehr wurde bislang vor dem Hintergrund der von Husak et al. und Kasper et al. (a.a.O.) präsentierten Daten angenommen, dass lediglich spezielle virale Konstrukte oder ganze Viruspartikel nach einer Injektion in das Gehirn in retinale Ganglienzellen transportiert werden. Ein Nucleinsäuremolekül ist allerdings in vielfältiger Hinsicht vorteilhaft gegenüber einem Viruspartikel oder viralem Konstrukt. So ist ein solches wesentlich einfacher herzustellen, zeichnet sich durch eine erhöhte Stabilität aus und ist sowohl als eigentliche Wirksubstanz oder auch als Trägermolekül für eine daran angekoppelte Wirksubstanz besonders geeignet. Besonders vorteilhaft ist bei der Verwendung eines Nucleinsäuremoleküls, dass dieses im Organismus keine virusspezifischen Nebenwirkungen induziert und deshalb als Therapeutikum besonders geeignet ist.

Oligonucleotide mit beliebigen Nucleotidsequenzen sind umfasst. Erfindungsgemäß werden deshalb unter einem Nucleinsäuremolekül lineare oder auch verzweigtkettige, durch 3', 5'-Phosphodiesterbindungen verknüpfte Di-, Tri- usw. -nucleotide verstanden. Derartige Nucleinsäuremoleküle lassen sich einfach durch zielgerichtete Nucleotidsynthese oder durch unvollständige enzymatische oder chemische Spaltung von Nucleinsäuren herstellen. Solche Verfahren sind im Stand der Technik allgemein bekannt und dem Fachmann geläufig; vgl. Sambrook, J. and Russell, D. W. (2001), "Molecular Cloning - A Laboratory Manual", Cold Spring Harbor Laboratory Press, New York.

Insbesondere wird erfindungsgemäß unter einem Nucleinsäuremolekül kein Viruspartikel oder auch virales Konstrukt, wie ein viraler Vektor, verstanden.

Die Erfinder haben in diesem Zusammenhang erkannt, dass es für die Erfindung nicht entscheidend ist, welche Nucleotidsequenz das Nucleinsäuremolekül aufweist, obgleich bestimmte Sequenzen ggf. Vorteile hinsichtlich der gewünschten therapeutischen oder diagnostischen Anwendung des Mittels haben können. Die Erfinder haben vielmehr ein allgemeines Prinzip erkannt und technisch ausgenutzt, mittels dem unabhängig von der Nucleotidsequenz des eingesetzten Nucleinsäuremoleküls zielgerichtet Erkrankungen der retinalen Ganglienzellen therapiert bzw. diagnostiziert werden können.

Ein zielgerichteter Transport in retinale Ganglienzellen bedeutet erfindungsgemäß, dass andere Zelltypen, insbesondere andere Zellschichten der Retina, weitgehend unberührt bleiben, d.h. eine Injektion des Nucleinsäuremoleküls in das Gehirn zum im Wesentlichen exklusiven Transport des letzteren in retinale Ganglienzellen führt. Dabei wird in Kauf genommen, dass ggf. geringe Mengen des Nucleinsäuremoleküls in nicht-RGC bzw. andere Zelltypen transportiert werden, diese jedoch im Vergleich zur Menge an Nucleinsäuremolekülen, die zielgerichtet in die RGC transportiert werden, äußerst gering ist und deshalb im Hinblick auf die Vorteile tolerierbar sind.

Unter einem Mittel wird erfindungsgemäß eine jegliche Zusammensetzung verstanden, wie eine pharmazeutische Zusammensetzung bzw. ein Arzneimittel oder eine diagnostische Zusammensetzung sowie ein Stoff zur Verwendung in der Forschung, der entweder ausschließlich aus Nucleinsäuremolekül besteht oder ggf. zusätzlich eine Puffersubstanz, einen pharmazeutisch oder diagnostisch akzeptablen Träger oder auch zumindest einen weiteren Wirkstoff, diagnostischen Marker oder sonstigen Hilfsstoff aufweist.

Ein erfindungsgemäßes Mittel kann auch außerhalb eines Lebewesens verwendet werden, bspw. als wissenschaftliches Tool, das in isolierte Zell- oder Gewebekulturen, die retinale Ganglienzellen aufweisen, einbringbar bzw. injizierbar ist.

Erfindungsgemäß ist das Mittel zur Injektion in den *Colliculus superior* ausgebildet.

Der *Colliculus superior* ist ein Organ, das ein Teil des Mittelhirndachs bildet. Die zu diesem Organ ziehenden Nervenbahnen führen u.a. visuelle Signale und bilden in ihm Repräsentationen des visuellen Feldes aus.

Die Erfinder konnten überraschenderweise zeigen, dass ein Nucleinsäuremolekül enthaltendes derart ausgebildetes Mittel, wenn dieses in den *Colliculus superior* eines Säugetiergehirns injizierbar ist, nach der Injektion über retrograden Transport, d.h. von der Peripherie einer Nervenzelle in Richtung des Zellkörpers, exklusiv in retinale Ganglienzellen transportiert wird.

Mit dieser Maßnahme wird ein Mittel bereitgestellt, das in eine anatomisch gut zugängliche und lokalisierbare Struktur des Mittelhirns injizierbar ist, insbesondere ist für eine selektive Applikation des Mittels bzw. Nucleinsäuremoleküls in die retinalen Ganglienzellen keine gefährliche Injektion in das Auge erforderlich.

An das Nucleinsäuremolekül kann ein Wirkstoff gekoppelt sein.

Diese Maßnahme hat den besonderen Vorteil, dass das Nucleinsäuremolekül als Trägersubstanz, d.h. als so genannter Carrier, verwendet wird, und nahezu jeder beliebige Wirkstoff über eine entsprechende Ankopplung an das Nucleinsäuremolekül zu den retinalen Ganglienzellen transportiert werden kann und dort bspw. eine therapeutisch nutzbare Aktivität entfalten kann. Dadurch können auch solche Wirkstoffe in die retinalen Ganglienzellen transportiert werden, die sich bislang völlig unselektiv im Organismus verteilen und deshalb für eine zielgerichtete Therapie nicht nutzbar waren. Im Stand der Technik, vgl. Sambrook and Russell (a.a.O.), sind hinreichend Methoden beschrieben, wie ein Nucleinsäuremolekül, bspw. ein Oligonucleotid bestehend aus 10 bis 30 Nucleotiden, an eine beliebige Substanz gekoppelt werden kann. Dieser Komplex bzw. ein entsprechendes erfindungsgemäßes Mittel kann dann über eine feine Kanüle in den *Colliculus superior* injiziert werden, bspw. mittels stereotaktischer Injektionen.

Das Nucleinsäuremolekül ist selbst als Wirkstoff ausgebildet.

Diese Maßnahme hat den Vorteil, dass bspw. die im Stand der Technik etablierte Antisense-Technologie verwendet werden kann, um gezielt retinale Ganglienzellen zu manipulieren. Die Ankopplung eines weiteren Wirkstoffes ist somit überflüssig. Vielmehr lässt sich unter Heranziehung von Datenbanken, die Informationen über die Nucleotidsequenzen interessierender ggf. RGCspezifischer Gene oder mRNA-Moleküle enthalten, Nucleinsäuremoleküle konstruieren, die mit entsprechenden genetischer Information in den retinalen Ganglienzellen wechselwirken und dadurch bspw. die Genexpression in diesen Zellen inhibieren können. Dabei ist von Vorteil, dass, wie die Erfinder zeigen konnten, ein Nucleinsäuremolekül nach seiner Injektion in das Gehirn in den retinalen Ganglienzellen sowohl im Cytoplasma zu finden ist, dort bspw. mit mRNA-Molekülen wechselwirken kann, als auch im Nucleus zu finden ist und dort bspw. mit der genomischen DNA interagieren kann.

Das Nucleinsäuremolekül ist derart ausgestaltet, dass es die Genexpression in den retinalen Ganglienzellen inhibiert und eine Nucleotidsequenz aufweist, die weitgehend komplementär zu der Sequenz einer mRNA aus den retinalen Ganglienzellen ist.

Diese Maßnahme hat den Vorteil, dass ein solches Nucleinsäuremolekül unter stringenten Bedingungen mit einem entsprechenden komplementären Nucleinsäuremolekül hybridisiert, dadurch die Transkription der genomischen DNA bzw. die Translation des mRNA-Moleküls hemmt und letztlich zu einer Inhibition der Expression des kodierenden Gens führt. Dadurch lassen sich auf einfache und zielgerichtete Art und Weise Überexpressionen bestimmter Gene in retinalen Ganglienzellen verhindern, die mit der Erkrankung des Auges bzw. der Netzhaut assoziiert sind. Eine Vielzahl derartiger Gene ist mittlerweile sequenziert und die Sequenzen in öffentlich zugänglichen Datenbanken einsehbar. Anhand der Nucleotidsequenz der Gene lässt sich mittels Routinemaßnahmen ein Nucleinsäuremolekül konstruieren, das eine entsprechende weitgehend komplementäre Nucleotidsequenz aufweist und zur erfindungsgemäßen Verwendung geeignet ist.

Erfindungsgemäß bedeutet "weitgehend komplementär", dass das Nucleinsäuremolekül in der Lage ist, unter stringenten Bedingungen mit der mRNA aus den retinalen Ganglienzellen über Wasserstoffbrückenbindungen zu hybridisieren. Dies ist dann möglich, wenn das Nucleinsäuremolekül über hinreichend lange Abschnitte eine Nucleotidsequenz aufweist, die komplementär zu einer Nucleotidsequenz der mRNA ist. Nicht erforderlich ist allerdings, dass das Nucleinsäuremolekül über seine gesamte Länge mit der mRNA hybridisieren kann, d.h. über die gesamte Länge eine komplementäre Nucleotidsequenz aufweist. Insofern bedeutet weitgehend komplementär im Sinne der Erfindung auch, dass einzelne Nucleotide innerhalb der Nucleotidsequenz des Nucleinsäuremoleküls nicht mit der mRNA aus den retinalen Ganglienzellen Wasserstoffbrückenbindungen ausbilden können, das Nucleinsäuremolekül insgesamt jedoch in der Lage ist, mit der entsprechenden mRNA zu hybridisieren und damit die Genexpression zu modulieren. Die Hybridisierungseigenschaften eines Nucleinsäuremoleküls lassen sich durch Routinemaßnahmen, wie bspw. der Erstellung von Schmelzkurven, auf einfache Art und Weise feststellen. Ausgehend von den bekannten Nucleotidsequenzen der in ihrer Expression zu modulierenden Gene gelangt der Fachmann deshalb unter Zuhilfenahme seines Fachwissens und der Durchführung von Routineexperimenten zielgerichtet zu einem Nucleinsäuremolekül, das weitgehend komplementär zu der Sequenz einer mRNA aus den retinalen Ganglienzellen ist.

Durch diese bevorzugte Ausgestaltung kann bspw. ein Nucleinsäuremolekül bereitgestellt werden, mit dem die eingangs genannten Erkrankungen der Retina, nämlich Glaukom oder Retinopathia Diabetica, behandelt werden können. So ist nämlich bekannt, dass bei diesen Erkrankungen als auch bei ischämischen Schädigungen apoptotische Prozesse ablaufen, die letztlich zum Absterben der Ganglienzellen führen können. Der Apoptoseprozess wiederum wird durch die Aktivität diverser Proteine vermittelt, wie bspw. c-fos, c-jun, p53, Bax, Apaf1, Caspase 9, Caspase 3, Caspase 6 PARP. Ein Überblick über in die Apoptose der Ganglienzellen involvierte Proteine bzw. Gene findet sich in Nickells R. W., 2004, The molecular biology of ganglion cell death: caveats and controversies, Brain Research Bulletin 62, Seiten 439 bis 446. Die Sequenzen dieser Gene sind bekannt, so dass es für den Fachmann ein Leichtes ist, Nucleinsäuremoleküle mit Nucleotidsequenzen, die zu den Nucleotidsequenzen dieser Gene bzw. der mRNAs der Gene komplementär sind, herzustellen. Mittels derartiger Nucleinsäuremoleküle lässt sich die Expression der krankheitsvermittelnden Gene inhibieren. Derartige Nucleinsäuremoleküle stellen deshalb ein therapeutisch wertvolles Tool für die Behandlung von derartigen Erkrankungen der Retina dar.

Alternativ kann das Nucleinsäuremolekül als siRNA ausgebildet sein.

Derartige siRNA-Moleküle (engl.: small interfering RNA) stellen doppelsträngige Strukturen aus Ribonucleinsäure dar. siRNA-Moleküle sind gegenüber einfachen Antisense-Oligonucleotiden zur Inhibierung der Genexpression besser geeignet, da sie einen autokatalytischen posttranskriptionellen Prozess auslösen können, der als RNA-Interferenz (RNAi) bezeichnet wird und zu einer äußerst effektiven Stilllegung der Expression bestimmter Gene, dem so genannten "gene silencing", führt. In eine biologische Zelle eingebracht werden die siRNA-Moleküle zu einem sogenannten Ribonucleasekomplex, dem sogenannten "RNA induced silencing complex" (RISC), rekrutiert. Dieser Komplex ist über das siRNA-Molekül in der Lage, an im Wesentlichen zu diesem komplementäre Strukturen, wie der mRNA eines in retinalen Ganglienzellen transkribierten Genes, zu binden und diese durch die Endonucleaseaktivität des RISC zu degradieren. Dies führt im Ergebnis zur Inhibierung der Expression des entsprechenden Genes, das für die mRNA kodiert, die zu einem Teil des siRNA-Moleküls komplementär ist.

Es ist ferner bevorzugt, wenn das Nucleinsäuremolekül derart ausgestaltet ist, dass es an ein anderes Nucleinsäuremolekül bindet, das für Kynureninaminotransferase II (KAT II) oder Teilen hiervon kodiert.

Diese Maßnahme hat den Vorteil, dass ein Nucleinsäuremolekül bereitgestellt wird, das besonders gut nach der Injektion in das Gehirn eines Säugetieres in retinale Ganglienzellen transportiert wird. Die Erfinder haben nämlich ein Oligonucleotid mit der Modellnucleotidsequenz TTCATGTCTCTGCTGGTCGC konstruiert, wobei das 5'-Ende wie üblich auf der linken Seite und das 3'-Ende auf der rechten Seite zu finden ist, das nach Injektion in den *Colliculus superior* eines Rattenhirns zielgerichtet in retinale Ganglienzellen transportiert wird. Überraschend war in diesem Zusammenhang, dass ein solches Nucleinsäuremolekül in den retinalen Ganglienzellen über längere Zeit stabil vorliegt und dort die Expression von KAT II herunterreguliert.

Selbstverständlich kann ein solches oder ein vergleichbares Nucleinsäuremolekül erfindungsgemäß am 5'- bzw. 3'-Ende weitere Nucleotidsequenzen aufweisen, ohne dass dadurch der zielgerichtete Transport in die retinalen Ganglienzellen aufgehoben wird bzw. die Inhibition der Expression von KAT II deutlich reduziert wird.

Die Erfinder haben anhand eines Modellnucleinsäuremoleküls mit vorstehend beschriebener Nucleotidsequenz beispielhaft belegt, dass ausgehend von bekannten Nucleotidsequenzen, die für ein interessierendes Gen kodieren, eine unbegrenzte Anzahl verschiedenster Nucleinsäuremoleküle hergestellt werden kann, die in den *Colliculus superior* injizierbar sind, nach retrogradem Transport zielgerichtet in retinale Ganglienzellen gelangen und dort die Expression des entsprechenden Genes inhibieren oder ausschalten können.

Die Erfinder haben deshalb ein allgemein gültiges technisches Konzept entwickelt, das in der Bereitstellung eines therapeutisch oder diagnostisch nutzbaren Mittels besteht, das Nucleinsäuremoleküle enthält, mit dem in einem Lebewesen ohne größere operative Eingriffe auf schonende Art und Weise die Expression jedes beliebigen Genes exklusiv in retinalen Ganglienzellen moduliert bzw. inhibiert werden kann. Die Erfindung ist deshalb nicht auf ein bestimmtes Nucleinsäuremolekül begrenzt.

Nach einer bevorzugten Variante ist an das Nucleinsäuremolekül ein detektierbarer Marker gekoppelt.

Diese Maßnahme hat den besonderen Vorteil, dass ein Mittel bereitgestellt wird, mit dem sich bspw. im Rahmen der neurobiologischen Grundlagenforschung der retrograde Transport von Substanzen zu den retinalen Ganglienzellen untersuchen lässt.

Als Marker kommt eine jede Substanz in Frage, die mit bekannten Mitteln der Diagnostik oder Molekularbiologie, wie z.B. der Mikroskopie, dem fluorescence activated cell sorting (FACS), Western-, Northernblotting, der Autoradiographie etc. detektiert werden kann. Beispiele für solche Substanzen sind fluoreszierender Farbstoffe, wie Fluorogold, Cy3, oder ein Peptid bzw. Protein, wie Peroxidase, Biotin, Streptavidin, Avidin, alkalische Phosphatase etc.

Vor diesem Hintergrund ist Gegenstand der vorliegenden Erfindung auch ein Nucleinsäuremolekül zur Behandlung einer Augenerkrankung, die durch eine gesteigerte Genexpression in retinalen Ganglienzellen charakterisiert ist, welches die Nucleotidsequenz TTCATGTCTCTGCTGGTCGC aufweist.

Im Stand der Technik ist es bislang nicht gelungen, ein solches Molekül bereitzustellen, das die Behandlung einer solchen Erkrankung der Netzhaut ermöglicht.

Ein weiterer Gegenstand der vorliegenden Erfindung betrifft eine pharmazeutische Zusammensetzung, die das vorstehend genannte Nucleinsäuremolekül und einen pharmazeutisch akzeptablen Träger sowie ggf. weitere Hilfs- und Wirkstoffe aufweist.

Pharmazeutisch akzeptable Träger bzw. Hilfsstoffe sind im Stand der Technik umfangreich beschrieben; vgl. bspw. Kibbe A. (2000), "Handbook of Pharmaceutical Excipients", American Pharmaceutical Association and Pharmaceutical Press. Eine solche Zusammensetzung kann weitere Wirkstoffe aufweisen, die in Zusammenhang mit der Behandlung der Erkrankung der Netzhaut vorteilhaft sind und dem Fachmann bekannt sind.

Offenbart wird ferner ein Verfahren zur zielgerichteten Verabreichung eines Mittels in die retinalen Ganglienzellen (RGC) bei einem menschlichen oder tierischen Lebewesen, mit den Schritten: (a) Bereitstellung eines Mittels, das Nucleinsäuremoleküle aufweist, und (b) Injektion des Mittels in den *Colliculus superior* des Lebewesens, wobei die Nucleinsäuremoleküle als Oligonucleotide ausgestaltet sind, die die Genexpression in RGC inhibieren.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die vorliegende Erfindung wird nun anhand von Ausführungsbeispielen näher erläutert, die rein illustrativ sind und die Tragweite der vorliegenden Erfindung keinesfalls einschränken. Dabei wird Bezug genommen auf die beigefügten Figuren, in denen Folgendes zu sehen ist:

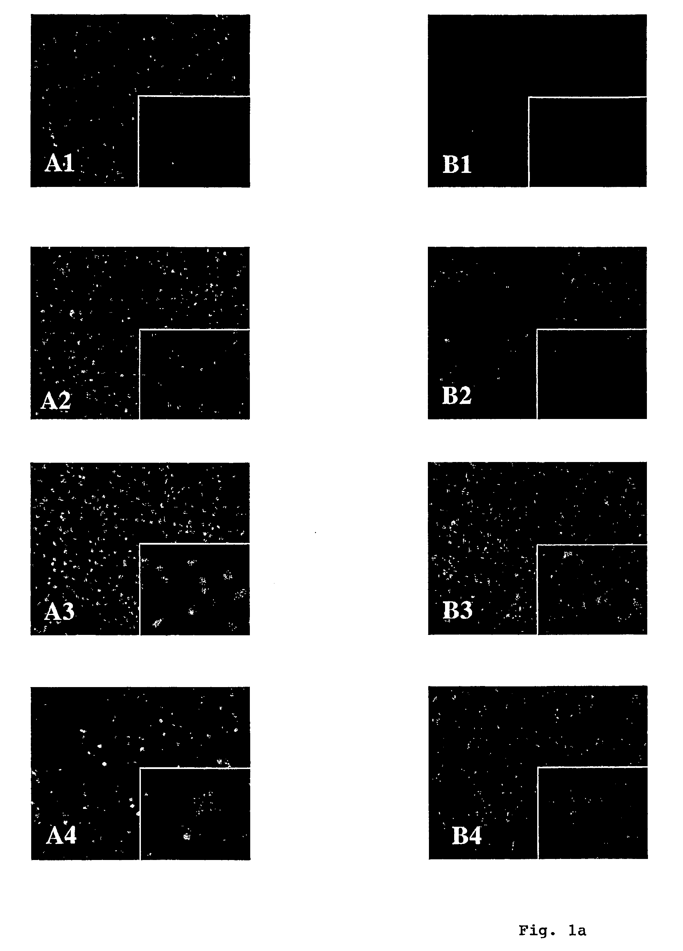

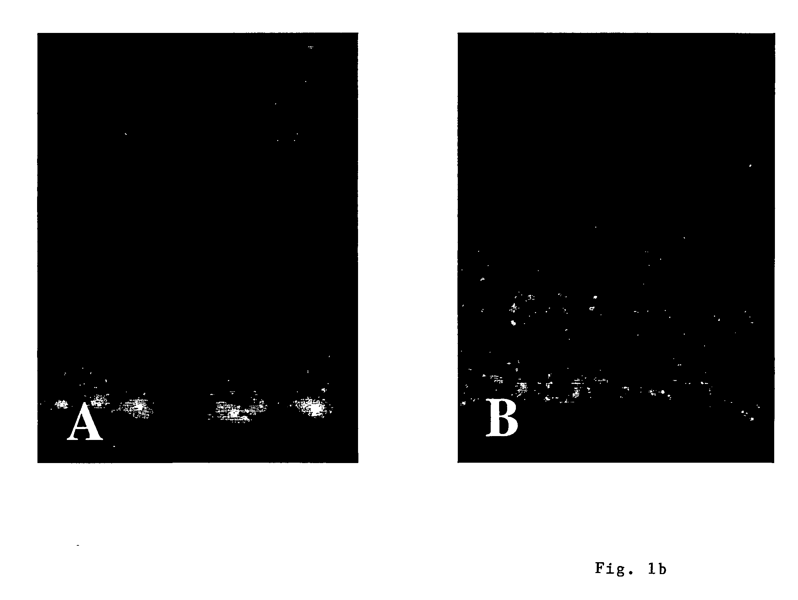

- Fig. 1: zeigt anhand von fluoreszenzmikroskopischen Untersuchungen an Retinaquerschnitten der Ratte den zielgerichteten Transport von Nucleinsäuremolekülen in retinale Ganglienzellen nach Injektion in den *Colliculus superior* und den unspezifischen Transport von Nucleinsäuremolekülen, die in den Glaskörper eines Rattenauges injiziert wurden;

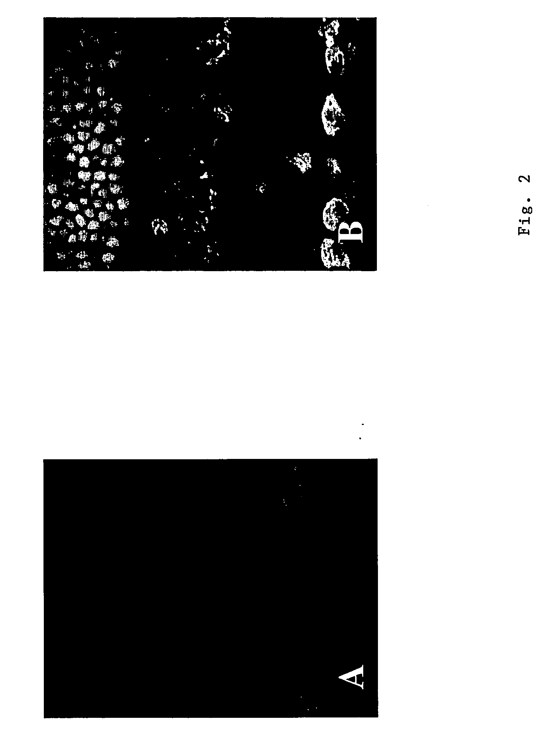

- Fig. 2: zeigt anhand von Untersuchungen an Retinaquerschnitten der Ratte mittels konfokaler Laserscanningmikroskopie den spezifischen Transport von Nucleinsäuremolekülen in retinale Ganglienzellen, die in den *Colliculus superior* injiziert wurden, wohingegen intravitreal injizierte Nucleinsäuremoleküle in diverse retinale Schichten transportiert werden;

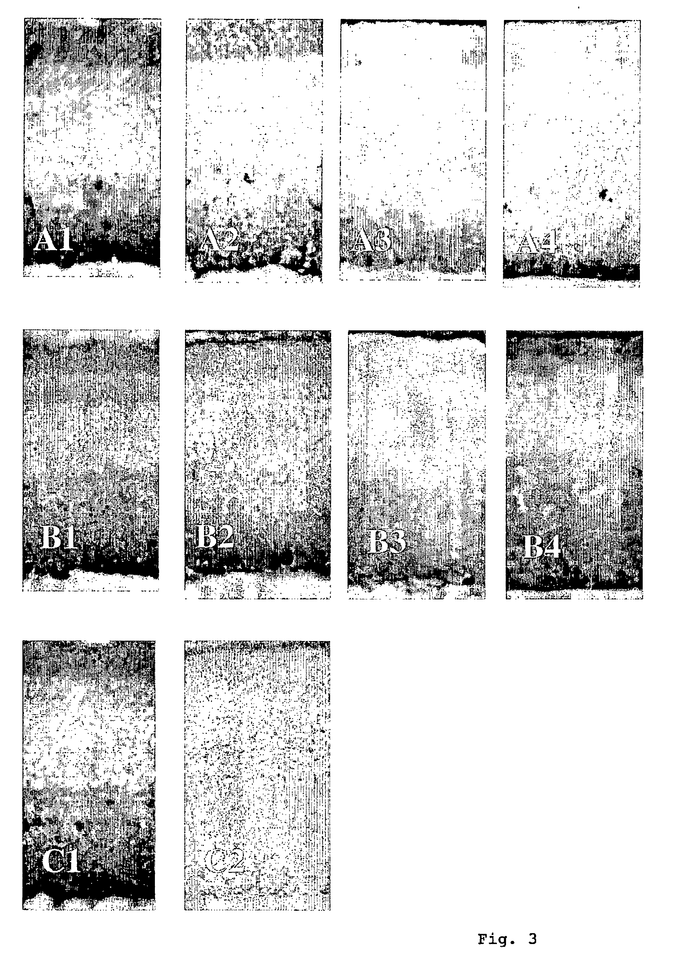

- Fig. 3: zeigt anhand von immunhistochemischen Untersuchungen an Retinaquerschnitten der Ratte die Inhibition der Genexpression von KAT II in retinalen Ganglienzellen durch ein spezifisches Nucleinsäuremolekül, das in den *Colliculus superior* injiziert wurde;

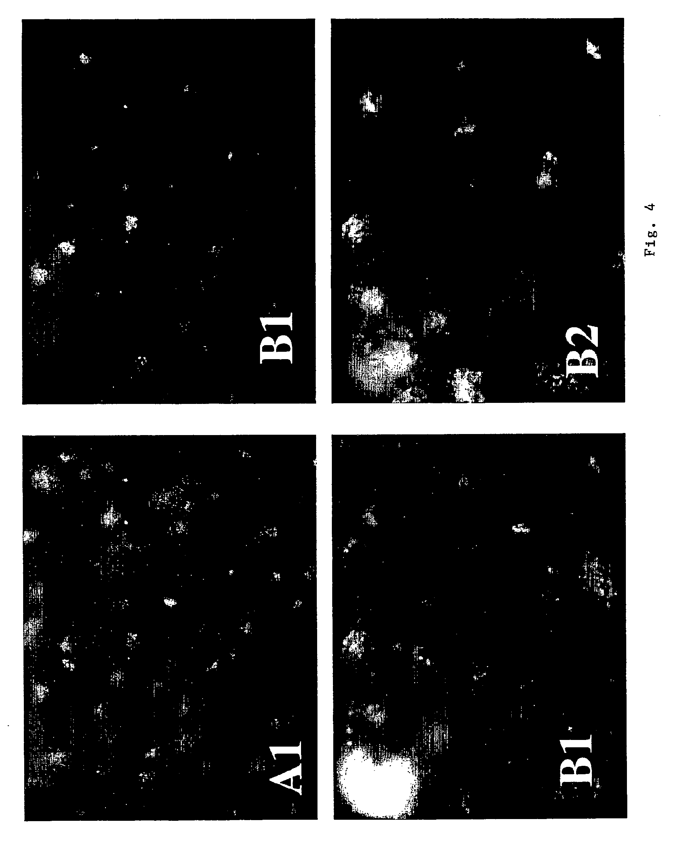

- Fig. 4: zeigt anhand von fluoreszenzmikroskopischen Untersuchungen an Flachpräparaten der Rattenretina eine CoFärbung von retinalen Ganglienzellen sowohl mit fluoreszenzmarkiertem Nucleinsäuremolekül als auch mit Fluorogold markiertem Nucleinsäuremolekül nach deren Injektionen in den *Colliculus superior.*

### Ausführungsbeispiele

### 1. MATERIAL UND METHODEN

### Tiere

Sämtliche Experimente wurden in Übereinstimmung mit den Richtlinien über die Behandlung von Tieren in der Europäischen Gemeinschaft und der ARVO (Association for Research in Vision and Ophthalmology) durchgeführt. Als Modelltiere wurden Brown-Norway-Ratten (Charles River, Wilmington, MA, Vereinigte Staaten von Amerika) mit einem Körpergewicht von 150 bis 200 g verwendet. Die Tiere wurden bei einem 12-Stunden Hell-Dunkel-Zyklus gehalten und nach Belieben mit Futter und Wasser versorgt.

### Nucleinsäuremoleküle

Die Nucleotidsequenz für die KAT II-mRNA wurde der GenBank-Datenbank entnommen; www.ncbi.nlm.nih.gov/Genbank/. Die Sequenz des Antisense-Oligonucleotides (ODN) gegen KAT II (KAT II ODN) lautete wie folgt: 5'-TTCATGTCTCTGCTGGTCGC-3'. Dieses als 20bp-Oligonucleotid ausgestaltete KAT II-ODN ist komplementär zum Bereich des Startcodons der KAT II-mRNA. Als Kontrolle wurde eine ODN mit der folgenden randomisierten Sequenz verwendet: 5'-GTACGTCTGTTCCTGTTCCG-3'. Die ODNs wurden kommerziell, ggf. als PS-ODN (PS: Phosphorothiotat-Rückgrat), synthetisiert (biomers.net, Ulm, Deutschland).

Fluoreszenzmarkierte oder unmarkierte PS-ODNs gegen KAT II, randomisierte ODN und NHS-Cy3-Fluoreszenzfarbstoff allein als Kontrolle wurden in ddH₂O (pH 7,4) mit einer Endkonzentration von 100 µM für intravitreale Injektionen und Injektionen in den *Colliculus superior* gelöst.

### Intravitreale Injektionen

Ratten wurden über eine intraperitoneale Injektion von Chloralhydrat (6 ml/kg Körpergewicht in einer 7%igen Lösung) anästhesiert. Die Injektion in die Augen erfolgte unter Verwendung einer hitzeausgezogenen Glaskapillare, die mit einer Mikrospritze (Drummond Scientific Co., Broomall, PA, Vereinigte Staaten von Amerika) verbunden war, bei direkter mikroskopischer Beobachtung. Tiere mit sichtbaren Schädigungen der Linsen wurden von den Experimenten ausgeschlossen.

Es wurde eine einzige Injektion von 2 µl mit 100 µM ODN bzw. PS-ODN (entspricht 200 pmol) verabreicht. Die kontralateralen Augen dienten als Kontrollaugen, in die die randomisierten Oligonucleotide injiziert wurden.

### Gewebepräparation

Für die Immunhistochemie wurden die Tiere mit CO₂ 2 Tage, 6 Tage, 2 Wochen und 6 Wochen nach den Injektionen getötet, und die Augen wurden unmittelbar enukleiert. Nach der Hemisektion der Augen entlang der *Ora serrata* wurden die Cornea, die Linsen und der Glaskörper entfernt. Die Augenkappen wurden für 30 Minuten in 4 % (Gewicht/Volumen) Paraformaldehyd in Phosphatpuffer (PB; 0,1 M pH 7,4) bei 4°C immersionsfixiert. Nach dreimaligem Waschen in PB wurden die Gewebe durch Immersion in 30 % (Gewicht/Volumen) Saccharose in PB über Nacht bei 4°C kryogeschützt. Die Proben wurden anschließend in eine Kryomatrix (Jung, Leica, Heidelberg, Deutschland) eingebettet.

Aus den eingebetteten Augenkappen wurden unter Verwendung eines Kryostats Radialschnitte mit 10 bis 12 µm Dicke herausgeschnitten, auf silanbeschichteten Objektträgern gesammelt, luftgetrocknet und für die weitere Verwendung bei 20°C gelagert.

### Immunhistochemie

Um reproduzierbare Ergebnisse zu erhalten, wurden die Experimente für jedes Setup mit Gruppen von 3 bis 4 Tieren durchgeführt. Die Schnitte wurden für 1 Stunde mit 20%igem Normal-Ziegenserum (NGS; Sigma, München, Deutschland) und 0,3 % Triton X-100 in phosphatgepufferter Saline (PBST) inkubiert, um die Hintergrundfärbung zu reduzieren. Die Primärantikörper wurden in PBST enthaltend 20 % NGS und 2 % Rinderserumalbumin (Sigma) verdünnt.

Die endogene Peroxidase wurde mit 3 % H₂O₂ in 40 % Methanol blockiert. Die Retinaschnitte wurden dann in einer Lösung enthaltend 10 % Normal-Ziegenserum und 0,3 % PBST inkubiert, um die Hintergrundfärbung zu reduzieren. Die Primärantikörper wurden 1:100 in PBST enthaltend 10 % NGS verdünnt und danach bei 4°C angewendet.

Um die KAT II-Immunreaktivität zu detektieren, wurde ein Kaninchenantikörper gegen KAT II verwendet (freundlicherweise bereitgestellt von Prof. Okuno, Wakayma, Japan).

Nach einer Inkubation über Nacht wurden am nächsten Tag die Schnitte mit PBS gespült (dreimal 5 Minuten), gefolgt von einer einstündigen Inkubation bei Raumtemperatur mit einem biotinkonjugierten Ziegen-Anti-Kaninchen-IgG-Sekundärantikörper (1:200; Vectastain Elite Kit, Vector Laboratories, Burlingame, CA, Vereinigte Staaten von Amerika) in PBST enthaltend 5 % NGS. Nach dem Spülen mit PBS wurden die Schnitte für 30 Minuten bei Raumtemperatur mit einem Avidin-Biotin-Peroxidase-Komplex (Vectastain Elite Kit) inkubiert, und die Färbung wurde unter Verwendung von Diaminobenzidin als Chromophor visualisiert. Bei den Kontrollschnitten wurde der Primärantikörper weggelassen. Die Schnitte wurden unter einem Olympus AX70-Mikroskop betrachtet.

### Injektion in den Culliculus superior

Die Tiere wurden tiefenanästhesiert und 7 µl des Fluoreszenzmarkers Hydroxystilbamidinmethansulfonat (Fluorogold, Molecular Probes, Eugene, OR, Vereinigte Staaten von Amerika) wurden über drei stereotaxische Injektionen in den *Culliculus superior* injiziert. 100 µM fluoreszenzmarkierte oder unmarkierte ODNs oder eine Kombination aus fluorogold- und fluoreszenzmarkierten ODNs wurden verwendet. 2 Tage, 6 Tage, 2 Wochen und 6 Wochen nach der Injektion wurden die Tiere mit CO₂ getötet. Die Augen wurden enukleiert, die Retinas herauspräpariert, Flachpräparate auf Cellulosenitratfiltern (Porengröße 60 µm; Sartorius, Long Island, NY, Vereinigte Staaten von Amerika) hergestellt und in 2 % Paraformaldehyd für 30 Minuten fixiert. Die Beobachtung erfolgte unmittelbar unter einem Fluoreszenzmikroskop. Aufnahmen wurden über ein digitales Bildgebungssystem, das an das Mikroskop angeschlossen war (ImagePro 3.0, Media Cybernetics Inc., Silver Spring, MD, Vereinigte Staaten von Amerika) erhalten, kodiert und analysiert.

### 2. ERGEBNISSE

### 2.1 Injektionen von Cy3-markierten PS-ODN

Nach intravitrealen Injektionen von Cy3-markierten ODNs oder Injektionen in den *Culliculus superior* wurde nach verschiedenen Zeitpunkten von einem Tag bis zwei Wochen die Transfektion der Retina und der retinalen Ganglienzellen unter Verwendung der Fluoreszenzmikroskopie an retinalen Flachpräparaten und Radialschnitten beobachtet.

### Retrograde Transfektion

Nach retrograden Injektionen von Cy3-markierten ODN in den *Culliculus superior* von Ratten wurden 1 Tag (Fig. 1a, A1), 2 Tage (Fig. 1a, A2), 6 Tage (Fig. 1a, A3) oder 2 Wochen (Fig. 1a, A4) nach der Injektion fluoreszenzmarkierte RGC in Retinaflachpräparaten analysiert. Dabei zeigte sich eine starke Färbung, die über die gesamte Zeit stark blieb. In Radialschnitten der Retina wurde auch nach bis zu 2 Wochen neben der RGC-Schicht keine Fluoreszenz in anderen Schichten der Retina beobachtet (Fig. 1b, A, 2 Tage) (andere Zeitpunkte sind nicht dargestellt).

Als' Kontrolle wurde der fluoreszierende Cy3-NHS-Ester allein (nicht an ODN gekoppelt) in den *Culliculus superior* injiziert. Dabei wurde nirgendwo in der gesamten Retina Fluoreszenz festgestellt (Daten nicht gezeigt).

Die in den *Culliculus superior* injizierten Nucleinsäuremoleküle wurden demnach hochselektiv und exklusiv in die RGC transportiert.

### Intravitreale Injektionen

Nach den intravitrealen Injektionen von Cy3-markierten PS-ODN in Ratten wurden in den Retinaflachpräparaten nach 1 Tag (Fig. 1a, B1), 2 Tagen (Fig. 1a, B2), 6 Tagen (Fig. 1a, B3) oder 2 Wochen (Fig. 1a, B4) fluoreszenzmarkierte Zellen in der RGC-Schicht detektiert. Tiefere Schichten der Retinaflachpräparate wurden ebenfalls angefärbt (Fig. 1a, B1 bis B4, die Aufnahmen zeigen den Fokus auf die RGC-Schicht). Bei der Untersuchung von Radialschnitten zeigte sich, dass auch Zellen anderer Schichten der Retina einschließlich des retinalen Pigmentepithels 2 Tage nach Injektion angefärbt waren (Fig. 1b, B). Dieses Muster blieb über den gesamten Zeitraum unverändert (andere Zeitpunkte werden nicht gezeigt).

Die in den Glaskörper der Augen injizierten Nucleinsäuremoleküle wurden demnach unspezifisch in eine Vielzahl von Geweben transportiert.

### 2.2 Laser-Scanning-Mikroskopie

Mittels Laser-Scanning-Mikroskopie ließen sich 4 Tage nach Durchführung der Injektionen nach beiden Methoden die fluoreszierenden ODNs sowohl im Cytoplasma als auch im Nucleus der RGC nachweisen. Nach den Injektionen in den *Culliculus* superior wurden ausschließlich RGC mit ODNs transfiziert (helle bzw. rote Färbung, Fig. 2, A). Nach intravitrealen Injektionen wurden sowohl RGC (hell bzw. rot) als auch andere Schichten der Retina mit ODNs transfiziert (Blaufärbung, Fig. 2b).

Die vorstehend beschriebenen Experimente werden deshalb bestätigt.

### 2.3 Immunhistochemie

Nach Injektionen von unmarkierten spezifischen KAT-II ODNs gemäß beiden Methoden wurde zu verschiedenen Zeitpunkten von bis zu einer Woche (Fig. 3) die Expression von KAT II in RGC untersucht. Dabei war bereits ein Tag nach der Injektion eine Runterregulation der KAT II-Expression zu beobachten (Fig. 3, A2), die 3 Tage nach der Injektion in den *Culliculus superior* das Maximum erreichte (Fig. 3, A3). Nach intravitrealen Injektionen wurde eine vergleichbare Runterregulation der KAT II-Expression beobachtet (Fig. 3, B2 bis B4). Die randomisierten ODN-Kontrollen (Fig. 3, A1, B1) zeigten eine Färbung, die der bei unbehandelten Ratten entspricht (Fig. 3, C1).

### 2.4 Co-Färbung von RGC mit fluoreszenzmarkierten ODN und Fluorogold

Nach Injektionen (intravitreal und in den *Culliculus superior,* verbunden mit retrograder Markierung der RGC mit Fluorogold FG) wurden nach monochromatischer Anregung von Cy3 (450 nm Wellenlänge) und FG (360 nm Wellenlänge) Aufnahmen gemacht (Fig. 4).

Nach Injektionen von Cy3-ODN in den *Culliculus superior* und der Markierung von RGC mit FG waren sämtliche retinalen Ganglienzellen (definiert als Zellen markiert mit FG (Fig. 4 A2)) gleichermaßen mit Cy3 gefärbt (Fig. 4 A1). Neben den FG-markierten RGC zeigten keine anderen Zellen irgendeine Cy3-positive Färbung. Das Muster war zu verschiedenen Zeitpunkten von 3 Tagen bis 2 Monaten vergleichbar (Daten nicht gezeigt). Die detektierte Cy3-Fluoreszenz war 3 Tage nach der Injektion am stärksten, 2 Wochen nach den Injektionen waren die detektierten Fluoreszenzsignale deutlich schwächer. Im Gegensatz hierzu blieb die FG-Fluoreszenz über den gesamten Zeitraum bei in etwa derselben Intensität (Daten nicht gezeigt).

Nach intravitrealen Injektionen von Cy3-ODN und Markierung von RGC mit FG waren sämtliche retinalen Ganglienzellen (definiert als Zellen markiert mit FG (Fig. 4, B2)) ebenso mit Cy3 markiert (Fig. 4, B1). Zusätzlich waren neben den RGC (FGmarkiert) weitere Zellen mit Cy3 gefärbt (Fig. 4, B1 verglichen mit B2). Dieses Muster blieb zu verschiedenen Zeitpunkten von 3 Tagen bis zu 2 Monaten unverändert (Daten nicht gezeigt).

### 3. FAZIT

Die Erfinder konnten zeigen, dass zur intravitrealen Injektion in das Säugetierauge ausgebildete Nucleinsäuremoleküle nach deren Injektion in sämtliche Zellschichten der Retina transportiert und darin akkumuliert werden, wohingegen zur Injektion in den *Culliculus superior* ausgebildete Nucleinsäuremoleküle nach deren Injektion exklusiv in die Zellschicht bestehend aus den retinalen Ganglienzellen transportiert bzw. darin akkumuliert werden. Die Erfinder konnten ferner zeigen, dass allein das entsprechende Nucleinsäuremolekül bzw. ein dieses enthaltendes Mittel nach Injektion in den *Culliculus superior* spezifisch in die retinalen Ganglienzellen transportiert wird, nicht jedoch ein gleichermaßen injizierter ungekoppelter Fluoreszenzfarbstoff.

Die Erfinder stellen folglich ein therapeutisch und diagnostisch äußerst wertvolles Tool bereit, mit dem Wirksubstanzen verschiedenster Art aber auch Diagnostika, die an das Nucleinsäuremolekül gekoppelt sind, über eine einfache Injektion in das Mittelhirn eines Patienten in die RGC appliziert werden können, wohingegen andere Zellen der Retina oder des Organismus weitgehend unbeeinflusst sind.

Ferner konnten die Erfinder demonstrieren, dass unter Verwendung von spezifischen Nucleinsäuremolekülen, bspw. Antisense-Oligonucleotiden oder siRNA-Molekülen, die in das Mittelhirn injizierbar sind, die Genexpression in den retinalen Ganglienzellen zielgerichtet moduliert werden kann. Das Nucleinsäuremolekül kann deshalb so ausgestaltet werden, dass dieses selbst therapeutisch wirksam ist.

## Patentansprüche

1. Verwendung eines Nucleinsäuremoleküls zur Herstellung eines Mittels zur Behandlung einer Erkrankung des Auges, die mit einer Genüberexpression in den retinalen Ganglienzellen (RGC) assoziiert ist, zur Injektion in den *Colliculus superior*, so dass das Nucleinsäuremolekül zielgerichtet in RGC transportiert wird, **dadurch gekennzeichnet, dass** das Nucleinsäuremolekül als Antisense-Oligonucleotid ausgestaltet ist, das die Genexpression in den RGC inhibiert und eine Nucleotidsequenz aufweist, die komplementär zu der Sequenz einer mRNA aus RGC ist.

2. Verwendung nach Anspruch 1, **dadurch gekennzeichnet, dass** die mRNA für ein Gen bzw. Protein kodiert, das ausgewählt ist aus der Gruppe bestehend aus: c-fos, c-jun, p53, Bax, Apafl, Caspase 9, Caspase 3, Caspase 6, PARP.

3. Verwendung nach Anspruch 1, **dadurch gekennzeichnet, dass** das Antisense-Oligonucleotid derart ausgebildet ist, dass es an ein Nucleinsäuremolekül bindet, das für Kynureninaminotransferase II (KAT II) oder Teilen hiervon kodiert.

4. Verwendung nach einem der Ansprüche 1 bis 3, **dadurch gekennzeichnet, dass** das Antisense-Oligonucleotid die Nucleotidsequenz TTCATGTCTCTGCTGGTCGC aufweist.

5. Verwendung nach einem der Ansprüche 1 bis 4, **dadurch gekennzeichnet, dass** an das Antisense-Oligonucleotid ein detektierbarer Marker gekoppelt ist.

6. Nucleinsäuremolekül zur Behandlung einer Augenerkrankung, die durch eine gesteigerte Genexpression in retinalen Ganglienzellen (RGC) charakterisiert ist, welches die Nucleotidsequenz TTCATGTCTCTGCTGGTCGC aufweist.

7. Pharmazeutische Zusammensetzung, die das Nucleinsäuremolekül nach Anspruch 6 und einen pharmazeutisch akzeptablen Marker sowie ggf. weitere Hilfs- und Wirkstoffe aufweist.

## Claims

1. Use of a nucleic acid molecule for the production of a composition for the treatment of a disease of the eye which is associated with a gene overexpression in the retinal ganglion cells (RGC), for the injection into the *Colliculus superior,* so that the nucleic acid molecule is transported into RGC in a targeted manner, **characterized in that** the nucleic acid molecule is configured as antisense oligonucleotide which inhibits the gene expression in the RGC and comprises a nucleotide sequence which is complementary to the sequence of an mRNA of RGC.

2. Use of claim 1, **characterized in that** the mRNA encodes a gene or protein, which is selected from the group consisting of: c-fos, c-jun, p53, Bax, Apafl, Caspase 9, Caspase 3, Caspase 6, PARP.

3. Use of claim 1, **characterized in that** the antisense oligonucleotide is configured in a way that it binds to a nucleic acid molecule which encodes kynurenin amino transferase II (KAT II) or parts thereof.

4. Use of any of claims 1 to 3, **characterized in that** the antisense oligonucleotide comprises the nucleotide sequence TTCATGTCTCTGCTGGTCGC.

5. Use of any of claims 1 to 4, **characterized in that** the antisense oligonucleotide is coupled to a detectable marker.

6. Nucleic acid molecule for the treatment of a disease of the eyes which is **characterized by** an increased gene expression in the retinal ganglion cells (RGC), which comprises the nucleotide sequence TTCATGTCTCTGCTGGTCGC.

7. Pharmaceutical composition which comprises the nucleic acid molecule of claim 6 and a pharmaceutically acceptable marker and, if applicable, further additives and active components.

## Revendications

1. Utilisation d'une molécule d'acide nucléique à la préparation d'un produit de traitement d'une affection oculaire qui est associée à une surexpression de gène dans les cellules ganglionnaires de la rétine (RGC), en vue de son injection dans le colliculus supérieur, de telle sorte que la molécule d'acide nucléique soit transportée de façon ciblée dans les RGC, **caractérisée en ce que** la molécule d'acide nucléique est configurée comme oligonucléotide antisens, qui inhibe l'expression du gène dans les RGC et qui présente une séquence de nucléotides qui est complémentaire à la séquence d'un ARN-m des RGC.

2. Utilisation selon la revendication 1, **caractérisée en ce que** l'ARN-m code pour un gène, respectivement une protéine, qui sont choisis parmi le groupe consistant en : c-fos, c-jun, p53, Bax, Apafl, caspase 9, caspase 3, caspase 6, PARP.

3. Utilisation selon la revendication 1, **caractérisée en ce que** l'oligonucléotide antisens est configuré de telle sorte qu'il s'attache à une molécule d'acide nucléique qui code pour la kynurénine aminotransférase Il (KAT II) ou pour des parties de celle-ci.

4. Utilisation selon l'une quelconque des revendications 1 à 3, **caractérisée en ce que** l'oligonucléotide antisens présente la séquence de nucléotides TTCATGTCTCTGCTGGCTCGC.

5. Utilisation selon l'une quelconque des revendications 1 à 4, **caractérisée en ce qu'**un marqueur détectable est accouplé à l'oligonucléotide antisens.

6. Molécule d'acide nucléique qui présente la séquence de nucléotides TTCATGTCTCTGCTGGCTCGC, pour le traitement d'une affection oculaire qui est **caractérisée par** une expression accrue de gène dans les cellules ganglionnaires de la rétine (RGC).

7. Composition pharmaceutique qui contient la molécule d'acide nucléique selon la revendication 6 et un marqueur pharmaceutiquement acceptable, ainsi que d'autres substances auxiliaires et actives.
